# FASCICULE DE BREVET EUROPEEN

(11) **EP 0 901 523 B1**
(45) Date de publication et mention de la délivrance du brevet: **21.02.2007**
(21) Numéro de dépôt: 97925119.6
(22) Date de dépôt: 22.05.1997
(51) Int. Cl.: C12N 5/10

(54) **LIGNEES DE CELLULES AVIAIRES IMMORTALISEES**
IMMORTALISIERTE GEFLÜGELZELLINIEN
IMMORTALIZED AVIAN CELL LINES

(30) Priorité: 23.05.1996 FR 9606629
(43) Date de publication de la demande: 17.03.1999
(73) Titulaire: INSTITUT NATIONAL DE LA RECHERCHE AGRONOMIQUE (INRA), 75338 Paris Cédex 07 (FR); CENTRE NATIONAL DE LA RECHERCHE SCIENTIFIQUE (CNRS), 75016 Paris (FR); ECOLE NORMALE SUPERIEURE DE LYON, 69364 Lyon Cedex 07 (FR); MERIAL, 69007 Lyon (FR)
(72) Inventeur: BOUQUET, Jean-François, F-69280 Sainte Consorce (FR); BENCHAIBI, Miloud, F-67000 Strasbourg (FR); SAMARUT, Jacques, F-69100 Villeurbanne (FR); DESMETTRE, Philippe, F-69130 Ecully (FR)
(74) Mandataire: Nargolwalla, Cyra
(86) Numéro de dépôt international: PCT/FR1997/000898
(87) Numéro de publication internationale: WO 1997/044444

(56) Documents cités:
- EP-A- 0 242 272
- WO-A-91/18971
- WO-A-92/10563
- WO-A-93/20200
- ONCOGENE, vol. 8, 1993, pages 619-624, XP002038734 GUILHOT ET AL: "THE 12S ADENOVIRAL E1A PROTEIN IMMORTALIZES AVIAN CELLS AND INTERACTS WITH THE AVIAN RB PRODUCT" cité dans la demande
- MOLECULAR AND CELLULAR BIOLOGY, vol. 7, no. 8, Août 1987, pages 2794-2802, XP002038735 NEUFELD ET AL: "IMMORTALIZATION OF HUMAN FIBROBLASTS TRANSFORMED BY ORIGIN-DEFECTIVE SIMIAN VIRUS 40" cité dans la demande
- JOURNAL OF VIROLOGY, vol. 67, no. 10, Octobre 1993, pages 6025-6032, XP002038736 NANICHE ET AL: "HUMAN MEMBRANE COFACTOR PROTEIN (CD46) ACTS AS A CELLULAR RECEPTOR FOR MEASLES VIRUS" cité dans la demande
- ONCOGENE, vol. 10, 1995, pages 1501-1509, XP002038737 ROSENBERG ET AL: "OVEREXPRESSION OF HUMAN CYCLIN A ADVANCES ENTRY INTO S PHASE" cité dans la demande
- NATURE, vol. 349, 14 Février 1991, pages 612-614, XP002038738 GREGORY ET AL: "ACTIVATION OF EPSTEIN-BARR VIRUS LATENT GENES PROTECTS HUMAN B CELLS FROM DEATH BY APOPTOSIS" cité dans la demande

## Description

La présente invention est relative à une lignée de cellules aviaires et ses dérivés.

L'établissement de lignées cellulaires à partir d'organes prélevés sur les espèces aviaires ne peut être obtenu spontanément, comme c'est le cas avec certains organes provenant d'espèces mammifères.

Les seules lignées cellulaires disponibles jusqu'à maintenant ont été obtenues en utilisant les propriétés transformantes de certains virus aviaires ayant des propriétés oncogènes, tels que les rétrovirus du groupe leucoses aviaires ou le virus de la maladie de Marek, ou de certaines molécules chimiques telles que la méthylcholanthrène et la diéthyl-nitrosamine.

Ces lignées cellulaires présentent, pour la plupart, un caractère de transformation important qui les rend impropres à la multiplication de virus vaccinaux.

Des auteurs se sont engagés sur une nouvelle voie consistant à introduire dans les cellules un vecteur ne présentant pas de caractère oncogène mais capable d'intégrer dans ces cellules un gène choisi pour sa capacité à induire l'immortalisation.

Les premiers essais ont été réalisés à l'aide de vecteurs intégrant des gènes de rétrovirus aviaires tels que erbA, erbB.

La demande de brevet français FR-A-2 596 770 propose un procédé d'immortalisation dans lequel on infecte une culture de cellules aviaires ou de mammifères avec un vecteur ou un système ne présentant pas de caractère oncogène pour lesdites cellules mais capable d'intégrer dans ces cellules un gène choisi parmi v-myb, v-ets et v-erbA. Des vecteurs appropriés peuvent être les virus AMV, E26 et XJ12, ce dernier étant un virus dérivé du virus AEV dans lequel le gène v-erB, oncogène, a été supprimé.

Dans la pratique, ces essais ont permis d'obtenir des lignées cellulaires établies à partir de cellules de la lignée hématopoïétique, mais n'ont pas donné les résultats escomptés pour les cellules d'embryon de poulet en culture adhérente telles que les fibroblastes ou les cellules épithéliales.

Des lignées de cellules aviaires de type myéloblastoïde (cellules sanguines) non transformées ont pu être obtenues à l'aide de l'oncogène myb (demande de brevet internationale WO91/18971).

Parallèlement, des auteurs ont proposé les gènes précoces t et T du virus simien SV40 pour immortaliser des cellules provenant de différents tissus de mammifères (D.S. Neufeld et al., Molecular and Cellular Biology, août 1987, 2794-2802, O. Kellermann et F. Kelly, Differentiation 1986, 32 · 74-81 et demande de brevet français FR-A-2 649 721).

La demande de brevet français FR-A-2 649 721 propose de son côté une méthode d'immortalisation conditionnelle qui serait utilisable pour tous les types cellulaires et dans toutes les espèces, l'objectif étant ici de remédier à l'inconvénient de la grande spécificité des voies classiques (limitation à des espèces et/ou à des types cellulaires particuliers) : transformation des cellules par un virus transformant (adénovirus, virus d'Epstein-Barr, certains papovavirus tels que le virus SV40 ou le virus du polyome ; par exemple, le virus SV40 est indiqué comme ne transformant que les cellules de rongeurs et les cellules humaines) ; transfection avec des constructions contenant un gène transformant lié à un promoteur viral ; transfection avec un gène transformant lié à un promoteur cellulaire. Le choix de cette demande de brevet se porte sur une construction associant un fragment d'ADN de la séquence régulatrice de la vimentine et un fragment d' ADN codant pour un gène immortalisant, qui peut être l'antigène T du virus SV40 sous le contrôle du promoteur, inductible, de la vimentine. Les espèces aviaires ne sont jamais mentionnées dans ce document.

Dans l'espèce aviaire, l'utilisation réelle de tels oncogènes viraux n'a jamais été décrite excepté l'utilisation de la forme 12S de la protéine E1A de l'Adénovirus 5 humain qui a permis d'immortaliser des cellules épithélioïdes de caille (Guilhot et al. (1993), Oncogene 8 : 619-624).

Contre toute attente, les inventeurs ont réussi à produire une lignée cellulaire aviaire, immortelle et non transformée.

De manière plus générale, les inventeurs ont trouvé qu'il était possible de préparer des lignées cellulaires aviaires immortelles et non transformées même à partir de cellules de tissus aviaires, c'est-à-dire autres que les cellules circulantes du sang ou hématopoïétiques.

La présente invention a donc pour objet des cellules aviaires immortelles non transformées, en particulier provenant de tissus aviaires, c'est-à-dire autres que des cellules sanguines ou hématopoïétiques, notamment fibroblastes et cellules épithéliales, par exemple d'embryons.

La présente invention a en particulier pour objet des cellules aviaires immortelles, non transformées, comprenant, intégré dans leur génome, le gène SV40 T+t sous la dépendance du promoteur MTI (métallothionéine I murin).

De préférence, elles intègrent aussi le promoteur SV40 fonctionnellement lié au gène de résistance à la néomycine.

De préférence, les cellules intègrent aussi au moins une séquence LTR. La séquence LTR peut être délétée comme décrit dans les exemples.

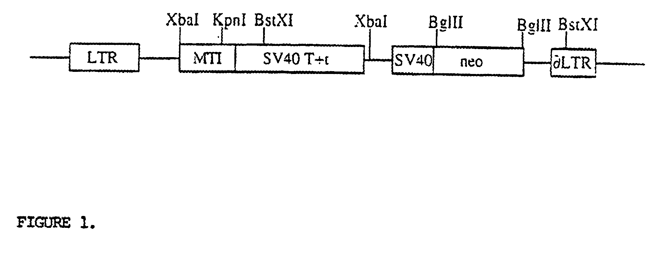

Les cellules intègrent de préférence le vecteur pDAMT visible à la figure 1.

Les cellules sont d'origine aviaire mais peuvent être en particulier issues de canard de barbarie.

L'invention a plus particulièrement pour objet la lignée cellulaire aviaire immortelle, non transformée, TDF-2A déposée à la CNCM (Collection Nationale de Cultures de Microoganismes de l'Institut Pasteur) sous la référence I-1712.

Bien entendu, l'invention couvre les cellules issues de ces lignées. Par cela, il faut comprendre que sont couvertes non seulement les cellules telles que déposées à la CNCM sous les références indiquées, mais aussi les cellules constituant la descendance des précédentes, d'une part celles obtenues par simple multiplication et pouvant subir des mutations lors des multiplications et d'autre part celles obtenues après modification volontaire, ce qu'on appelle alors les cellules dérivées, et bien sûr aussi celles ayant subi les deux types de modification.

L'invention couvre donc aussi les cellules dérivées obtenues par modifications des cellules ci-dessus. Ces modifications peuvent comprendre :
- Insertion d'une ou plusieurs cassettes d'expression comprenant chacune une ou plusieurs séquences nucléotidiques codant pour une molécule d'intérêt industriel, ces cassettes d'expression étant aptes à produire cette molécule après insertion dans les cellules de l'invention. La technique est parfaitement connue de l'homme du métier. Comme molécules d'intérêt industriel, on peut citer notamment des sous-unités virales de type peptide, protéine, glycoprotéine, notamment à usage de vaccin ou de réactif de diagnostic, des molécules protéiques telles que des hormones, etc.
- Infection chronique par un virus apte à se multiplier dans ces cellules, à des fins de production virale ou de vaccin, avec ou sans modification préalable de la sensibilité vis-à-vis de ce virus. L'infection peut aussi ne pas être chronique mais réalisée sur un lot de cellules choisi pour la multiplication virale.
   (Les modifications qui suivent s'entendent de préférence et avantageusement combinées aux deux types précédents).
- Introduction de gènes de survie ou anti-apoptose autres que bcl-2 tels que les gènes codant pour les protéines p19E1B de l'adénovirus humain (Rao et al. (1992), Proc. Natl. Acad. Sci. USA 89 : 7742-7746), LMP-1 (Gregory et al. (1991), Nature 349 : 612-614) et BHRF1 (Pearson et al. (1987), Virology 960 : 151-161) du virus Epstein Barr, ICP34.5 du virus herpes simplex (Chou et Roizman (1992), Proc. Natl. Acad. Sci. USA 89 : 3266-3270) et p35 du baculovirus (Clem et al. (1991), Science 254 : 1388-1390), afin de rendre ces lignées plus résistantes aux conditions de culture, notamment maintien à confluence.
- Surexpression de gènes impliqués dans le contrôle du cycle cellulaire par des vecteurs appropriés pour augmenter la vitesse de prolifération. En effet, il a été montré que, dans certains cas, la surexpression de gènes codant pour des cyclines entraînait un raccourcissement du cycle cellulaire et donc une augmentation de la vitesse de prolifération (Rosenberg et al. (1995), Oncogéne 10 : 1501-1509 ; Quelle et al. (1993), Genes and Dev. 7 : 1559-1571).
- Modification du spectre de sensibilité virale des lignées par intégration de gènes codant pour des récepteurs de virus d'intérêt, en vue de leur multiplication.
   On peut se référer à l'espèce mammifère où l'expression du récepteur du virus de la rougeole (CD46) par des cellules murines, normalement non permissives au virus, entraîne une sensibilité de ces cellules à ce virus et la capacité à le répliquer (Naniche et al. (1993), J. Virol. 67 : 6025-6032). L'intérêt est notamment de rendre les cellules sensibles à un virus afin de le produire sur celles-ci.
- Intégration d'oncogènes aptes à accélérer la croissance cellulaire.

Il va de soi que les cellules dérivées selon l'invention peuvent comprendre une ou plusieurs des modifications présentées ci-dessus.

L'invention a encore pour objet un procédé de production de molécules d'intérêt industriel ou de virus, comprenant la culture des cellules décrites ci-dessus.

Dans le cadre de la présente invention, on s'oriente notamment vers la production de molécules ou de virus pour la réalisation de réactifs de diagnostic ou de vaccins, ou encore de molécules d'intérêt thérapeutique.

L'invention va être maintenant décrite plus en détail à l'aide d'un mode de réalisation pris à titre d'exemple non limitatif et se référant à la figure 1, qui montre la structure du vecteur pDAMT servant à préparer la lignée TDF-2A, avec :
LTR : séquence répétée directe (Long Terminal Repeat)
LTR : LTR délétée
MTI : promoteur Metallothionéine I murin
SV40 T+t : région précoce SV40
SV40 : promoteur SV40

### EXEMPLE 1 = Production de la lignée cellulaire TDF-2A

### 1. Description de son origine et de ses caractéristiques

### 1.1 Description du vecteur utilisé : vecteur pDAMT

Il comporte la région précoce du virus SV40 (code pour les antigènes T et t) (fragment HindIII/BamHI) (Fiers et al.(1978), Nature 273 : 113-120) sous le contrôle du promoteur métallothionéine I de souris (fragment EcoRI/BgIII transformé en site HindIII) (Durnam et al.(1980), Proc. natl. Acad. Sci. USA 77 : 6511-6515 ; Brinster et al. (1982), Nature 296 : 39-42).

Le fragment EcoRI/EcoRI contenant cette unité transcriptionnelle provenant du vecteur pMTSVneo (Peden et al. (1989), Exp. Cell. Res. 185 : 60-72) a été inséré dans le site Xbal du vecteur pDA1 (Aubert et al. (1991), J. Cell. Biol. 113 : 497-506). Ce dernier dérive essentiellement du génome du virus associé au sarcome de Rous-2 (RAV-2) après modification de la LTR située en 3'. En effet, la région U3 de la LTR 3' de RAV-2 a été délétée et liée aux régions R et U5 isolées de la LTR du virus associé au sarcome de Rous-1 (RAV-1). II porte également une unité transcriptionnelle contenant le gène de résistance à la néomycine sous le contrôle du promoteur SV40 dérivant du vecteur pSV2neo (Southern et Berg (1982), J. Mol Appl. Genet. 1 : 327-341). Voir figure 1.

### 1.2. Etablissement de la lignée et démonstration du caractère immortalisé

Des cellules provenant d'embryons de canard de Barbarie de 14 jours ont été transfectés par le vecteur pDAMT par la méthode utilisant le diméthylsulfoxide (DMSO) et décrite par Kawai et Nishizawa (1984), Mol. Cell. Biol. 4 : 1172-1174. Les cellules transfectées sont ensuite sélectionnées par application de généticine G418 (150 µg/ml) pendant 15 jours. Les clones résistants sont alors subcultivés régulièrement à raison de 1 à 2 passages par semaine. Après cette période de prolifération active de 3 mois, les cellules sont entrées dans une période de crise durant laquelle la plupart des cellules sont mortes. Après cette période qui a duré environ 2 mois, plusieurs clones ont repris une prolifération active suggérant leur immortalisation,

La lignée cellulaire TDF-2A est issue ainsi de 2 cultures.
Elle a été étudiée de manière plus approfondie.

Les cellules TDF-2A ont atteint 200 passages soit environ 460 générations et ont été ainsi maintenues en culture pendant plus de 600 jours en continu Comparativement, des cellules témoins, n'exprimant pas la région précoce du virus SV40 ne peuvent être maintenus en culture plus de 20 passages.

### 1.3. Caractéristiques de prolifération.

Les cellules immortalisées sont cultivées à 38°C, en flacon roulant, dans un milieu contenant du HAM F-10 10X à 6%, 199 HANKS 10X à 4%, Tryptose Broth Phosphate 2,95 % à 4%, Bicarbonate de sodium 5,6% à 2,5%, vitamine BME 100X à 0,1%, sérum de veau foetal à 3%, Kanamycine 5% à 1%, Vancomycine 0,5% à 1 %.

Dans ces conditions, leur taux de doublement est de 1 par 24 heures.

### 1.4. Expression de l'antigène T.

Par immunofluorescence ou immunophosphatase indirectes en utilisant un anticorps spécifique de l'antigène T (Pab 101 : Santa Cruz Biotechnology ref. sc147), il a été vérifié que toutes les cellules expriment l'antigène T dans leur noyau, indiquant qu'elles ont toutes intégré le vecteur.

Cette intégration a d'ailleurs été montrée par Southern-blot. L'ADN génomique des fibroblastes immortalisés a été digéré par les enzymes de restriction Xbal, BstXl. L'hybridation avec une sonde spécifique de l'antigène T (fragment Ndel/Ndel de 1018 pb) a permis de vérifier que l'unité transcriptionnelle permettant l'expression du gène immortalisant, insérée dans les cellules TDF-2A, n'avait pas subi de remaniements majeurs. En effet, la taille des fragments d'hybridation obtenus est conforme à celle attendue.

### 1.5. Absence de pouvoir tumorigène.

Les cellules immortalisées ne présentent pas de pouvoir tumorigène. Elles sont incapables de former des colonies en milieu semi-solide ou de former des tumeurs sur membrane chorioallantoïdienne d'oeufs de poule ou de cane. Elles sont également incapables de former des tumeurs sur souris nue (souris "nude"), sur poussins et canetons SPF (exempts d'organismes pathogènes) de 1 jours.

### 1.6. Caryotype.

Le caryotype des cellules TDF-2A a été étudié aux 114ème et 135ème passages. Il a permis de vérifier que les cellules étaient bien d'origine aviaire avec la présence de microchromosomes caractéristiques de cette espèce. De plus, les chromosomes observés sont représentatifs des chromosomes rencontrés dans les cellules primaires d'embryons de canard confirmant ainsi l'origine de la lignée.

### II. Propriétés.

Les cellules TDF-2A présentent notamment une sensibilité aux virus spécifiques du canard tels que l'adénovirus, le parvovirus et le reovirus qui sont habituellement répliqués sur cellules primaires d'embryons de canard. On peut donc produire ces virus sur cette lignée.

### EXEMPLE 2 : Caractérisation de la lignée TDF-2A par identification des sites d'intégration.

L'ADN génomique des cellules TDF-2A, préparé à partir des cellules provenant du 114ième et du 135ième passages, a été digéré par les enzymes de restriction BgIII et KpnI. L'ADN ainsi traité est alors soumis à une électrophorèse sur gel, suivie d'un transfert sur membrane de nylon, puis hybridé avec une sonde spécifique de l'antigène T (fragment Ndel/Ndel de 1018 pb). Ainsi, la digestion par BgIII permet d'obtenir deux bandes d'hybridation de haute taille (d'environ 15 et 23 kb) suggérant l'existence de deux sites d'intégration. La digestion par Kpnl entraîne l'obtention d'une bande majoritaire de haute taille (environ 20 kb) et d'au moins une bande minoritaire confirmant l'existence d'au moins deux sites d'intégration.

### EXEMPLE 3 : Multiplication de l'adenovirus V127 sur cellules TDF-2A.

Les cellules TDF-2A sont ensemencées en flacon roulant. L'adenovirus de la maladie des oeufs hardés souche V127 est inoculé à la mise en culture des cellules. Au bout de 6 jours, la récolte est effectuée par agitation de façon à décoller le tapis cellulaire. Celle-ci est donc composée du tapis cellulaire et de surnageant de culture. L'ensemble est homogénéisé par un traitement au broyeur de cellules ou homogénéiseur tel que Ultraturrax pendant 1 min à 13 500 tours/min (appareil IKA type T25).

La détermination du titre viral infectieux est réalisée en microméthode sur plaque de 96 puits. Les dilutions de virus sont inoculées sur tapis établi de cellules secondaires d'embryons de canard de Barbarie SPF. Chaque dilution virale est inoculée sur 6 cupules. Les plaques sont placées en incubation dans un incubateur à CO₂ pendant 8 jours. La présence de virus dans les cupules est contrôlée au microscope en observant l'effet cytopathique (ECP) caractéristique. Le titre infectieux est calculé selon la méthode de KARBER et est exprimé par le logarithme de l'inverse de la dilution virale donnant 50% d'ECP [Titre = d+r/Nx(n+N/2)] avec d égal à la dilution exprimée en log où il y a 100% de cupules positives, r égal à la raison de la dilution, N égal au nombre de cupules par dilution et n égal au nombre de cupules positives entre 0 et 100%).

La présence de virus est également confirmée par la recherche de l'activité hémagglutinante du surnageant viral à l'aide d'une suspension de globules rouges de poulet. Dans ce cas, 50 µl du surnageant des cupules précédentes sont déposés sur une plaque de microtitration Dynatech et 25 µl d'une suspension d'hématies de poule à 15.10⁶ cellules/ml sont ajoutés par cupule. Après agitation et 45 min d'incubation à température ambiante, sont considérées comme positives, toutes les cupules présentant une hémagglutination nettement visible. Le calcul du titre est réalisé également selon la méthode de KARBER.
Résultats : Les titres viraux obtenus sont équivalents à ceux obtenus sur cellules primaires d'embryons de canard.

## Revendications

1. Procédé de production de virus, comprenant la culture de cellules aviaires immortelles mais non transformées, comprenant intégré dans leur génome le gène SV40 T+t.

2. Procédé de production de virus selon la revendication 1, comprenant l'infection avec un virus de cellules aviaires immortelles mais non transformées, et la multiplication de ce virus sur cette cellule.

3. Procédé selon l'une des revendications 1 ou 2, **caractérisé en ce que** les cellules aviaires immortelles mais non transformées sont obtenues à partir de cellules de tissus aviaires.

4. Procédé selon l'une quelconque des revendications 1 à 3, **caractérisé en ce que** les cellules aviaires immortelles mais non transformées proviennent de fibroblastes ou de cellules épithéliales.

5. Procédé selon l'une quelconque des revendications 1 à 4, **caractérisé en ce que** le gène SV40 T+t est sous la dépendance du promoteur métallothionéine I.

6. Procédé selon la revendication 5, **caractérisé en ce que** les cellules aviaires immortelles mais non transformées intègrent aussi le promoteur SV40 fonctionnellement lié au gène de résistance à la néomycine.

7. Procédé selon l'une des revendications 5 ou 6, **caractérisé en ce que** les cellules aviaires immortelles mais non transformées intègrent aussi au moins une séquence LTR.

8. Procédé selon l'une quelconque des revendications 1 à 7, **caractérisé en ce qu'**elles contiennent en outre au moins une cassette d'expression comprenant au moins une séquence nucléotidique codant pour une sous-unité virale de type peptide, protéine, glycoprotéine.

9. Procédé selon l'une quelconque des revendications 1 à 8, **caractérisé en ce qu'**elles sont en outre infectées, de préférence chroniquement, par un virus apte à se multiplier dans ces cellules.

10. Procédé selon l'une quelconque des revendications 1 à 9, **caractérisé en ce que** les cellules aviaires immortelles mais non transformées contiennent en outre un gène anti-apoptose autre que bcl-2, de préférence choisi parmi le groupe consistant en p19E1B de l'adénovirus humain, LMP-1 du virus Epstein Barr, BHRF1 du virus Epstein Barr, ICP34.5 du virus herpes simplex et p35 du baculovirus.

11. Procédé selon l'une quelconque des revendications 1 à 10, **caractérisé en ce que** les cellules aviaires immortelles mais non transformées intègrent en outre des vecteurs aptes à sur exprimer un ou des gènes impliqués dans le contrôle du cycle cellulaire afin d'augmenter la vitesse de prolifération.

12. Procédé selon l'une quelconque des revendications 1 à 11, **caractérisé en ce que** les cellules aviaires immortelles mais non transformées intègrent en outre des gènes codant pour des récepteurs viraux.

13. Procédé selon l'une quelconque des revendications 1 à 12, **caractérisé en ce que** les cellules aviaires immortelles mais non transformées intègrent en outre des oncogènes aptes à accélérer la croissance cellulaire.

14. Cellules aviaires immortelles mais non transformées comprenant, intégré dans leur génome, le gène SV40 T+t sous la dépendance du promoteur métallothionéine I.

15. Lignée cellulaires aviaires immortelle, non transformée, TDF-2A déposée à la CNCM Collection Nationale de Cultures de Microorganismes de l'Institut Pasteur sous la référence I-1712.

16. Procédé de production de virus, **caractérisé en ce qu'**un virus spécifique du canard, tel que adénovirus, parvovirus et réovirus du canard, est répliqué sur la lignée TDF-2A de la revendication 15 ou sur des cellules aviaires immortelles issues par multiplication de la lignée TDF-2A.

## Claims

1. A method for producing virus, comprising culture of immortal but non-transformant avian cells, comprising the SV40 T+t gene integrated into their genome.

2. A method for producing virus according to claim 1, comprising infecting immortal but non-transformant avian cells with a virus and multiplying said virus on said cell.

3. A method according to claim 1 or claim 2, **characterized in that** the immortal but non-transformant avian cells are obtained from avian tissue cells.

4. A method according to any one of claims 1 to 3, **characterized in that** the immortal but non-transformant avian cells derive from fibroblasts or epithelial cells.

5. A method according to any one of claims 1 to 4, **characterized in that** the SV40 T+t gene is under the dependency of the metallothionine I promoter.

6. A method according to claim 5, **characterized in that** the immortal but non-transformant avian cells also integrate the SV40 promoter, functionally linked to the neomycin resistance gene.

7. A method according to claim 5 or claim 6, **characterized in that** the immortal but non-transformant avian cells also integrate at least one LTR sequence.

8. A method according to any one of claims 1 to 7, **characterized in that** they further contain an expression cassette comprising at least one nucleotide sequence coding for a peptide, protein or glycoprotein type viral sub-unit.

9. A method according to any one of claims 1 to 8, **characterized in that** they are also infected, preferably chronically, by a virus which can multiply in said cells.

10. A method according to any one of claims 1 to 9, **characterized in that** the immortal but non-transformant avian cells further contain an anti-apoptosis gene other than bcl-2, preferably selected from the group consisting of human adenovirus p19E1B, Epstein Barr virus LMF-1, Epstein Barr virus BHRF1, herpes simplex virus ICP34.5 and baculovirus p35.

11. A method according to any one of claims 1 to 10, **characterized in that** the immortal but non-transformant avian cells further include vectors which can over-express one or more genes involved in control of the cell cycle, to enhance the proliferation rate.

12. A method according to any one of claims 1 to 11, **characterized in that** the immortal but non-transformant avian cells further integrate genes coding for viral receptors.

13. A method according to any one of claims 1 to 12, **characterized in that** the immortal but non-transformant avian cells further include oncogenes which can accelerate cell growth.

14. Immortal but non-transformant avian cells comprising, integrated into their genome, the SV40 T+t gene under the dependence of the metallothionine I promoter.

15. TDF-2A immortal but non-transformant avian cells filed at the CNCM, Collection Nationale de Cultures de Microorganismes [National Microorganism Culture Collection] by the Institut Pasteur with accession number I-1712.

16. A method for producing a virus, **characterized in that** a specific duck virus such as duck adenovirus, parvovirus or reovirus, is replicated on the TDF-2A line of claim 15 or on immortal avian cells derived by multiplication of the TDF-2A line.

## Patentansprüche

1. Verfahren zur Herstellung eines Virus, umfassend das Züchten immortaler aber nicht transformierter Vogelzellen, die in ihrem Genom integriert das Gen SV40 T+t enthalten.

2. Verfahren zur Herstellung eines Virus gemäß Anspruch 1, umfassend die Infektion immortaler aber nicht transformierter Vogelzellen mit einem Virus, und die Vermehrung dieses Virus auf dieser Zelle.

3. Verfahren nach einem der Ansprüche 1 oder 2, **dadurch gekennzeichnet, dass** die immortalen aber nicht transformierten Vogelzellen ausgehend von Zellen aviären Gewebes erhalten werden.

4. Verfahren nach einem der Ansprüche 1 bis 3, **dadurch gekennzeichnet, dass** die immortalen aber nicht transformierten Vogelzellen von Fibroblasten oder epithelialen Zellen abstammen.

5. Verfahren nach einem der Ansprüche 1 bis 4, **dadurch gekennzeichnet, dass** das Gen SV40 T+t vom Metallothionein-I-Promotor abhängig ist.

6. Verfahren nach Anspruch 5, **dadurch gekennzeichnet, dass** die immortalen aber nicht transformierten Vogelzellen auch den Promotor SV40 einschließen, der funktionell mit dem Neomycinresistenz-Gen verbunden ist.

7. Verfahren nach einem der Ansprüche 5 oder 6, **dadurch gekennzeichnet, dass** die immortalen aber nicht transformierten Vogelzellen auch mindestens eine LTR-Sequenz einschließen.

8. Verfahren nach einem der Ansprüche 1 bis 7, **dadurch gekennzeichnet, dass** die Zellen ferner mindestens eine Expressionskassette enthalten, umfassend mindestens eine Nucleotidsequenz, die eine virale Untereinheit vom Typ eines Peptids, Proteins oder Glykoproteins codiert.

9. Verfahren nach einem der Ansprüche 1 bis 8, **dadurch gekennzeichnet, dass** die Zellen ferner mit einem Virus infiziert sind, vorzugsweise chronisch, der fähig ist, sich in diesen Zellen zu vermehren.

10. Verfahren nach einem der Ansprüche 1 bis 9, **dadurch gekennzeichnet, dass** die immortalen aber nicht transformierten Vogelzellen ferner ein Antiapoptose-Gen enthalten, das nicht bcl-2 ist, vorzugsweise ausgewählt aus der Gruppe bestehend aus p19E1B des menschlichen Adenovirus, LMP-1 des Epstein-Barr-Virus, BHRF1 des Epstein-Barr-Virus, lCP34.5 des Herpes-simplex-Virus und p35 des Baculovirus.

11. Verfahren nach einem der Ansprüche 1 bis 10, **dadurch gekennzeichnet, dass** die immortalen aber nicht transformierten Vogelzellen ferner Vektoren einschließen, die fähig sind, ein oder mehrere an der Kontrolle des Zellzyklus beteiligte Gene überzuexprimieren, um die Proliferation zu beschleunigen.

12. Verfahren nach einem der Ansprüche 1 bis 11, **dadurch gekennzeichnet, dass** die immortalen aber nicht transformierten Vogelzellen ferner Gene enthalten, die virale Rezeptoren codieren.

13. Verfahren nach einem der Ansprüche 1 bis 12, **dadurch gekennzeichnet, dass** die immortalen aber nicht transformierten Vogelzellen ferner Onkogene enthalten, die fähig sind, das Zellwachstum zu beschleunigen.

14. Immortale aber nicht transformierte Vogelzellen, die, integriert in ihr Genom, das vom Metallothionein-I-Promotor abhängige Gen SV40 T+t umfassen.

15. Immortale, nicht transformierte aviäre Zelllinie TDF-2A, die bei der Hinterlegungsstelle für Mikroorganismen CNCM, Institut Pasteur, unter der Hinterlegungsnummer I-1712 hinterlegt ist.

16. Verfahren zur Herstellung eines Virus, **dadurch gekennzeichnet, dass** ein für Ente spezifischer Virus, wie Adenovirus, Parvovirus und Reovirus der Ente, auf der Linie TDF-2A gemäß Anspruch 15 oder auf immortalen Vogelzellen, die durch Vermehrung aus der Linie TDF-2A gewonnen wurden, repliziert wird.
